# EUROPEAN PATENT SPECIFICATION

(11) **EP 1 820 506 B1**
(45) Date of publication and mention of the grant of the patent: **26.03.2008**
(21) Application number: 06250764.5
(22) Date of filing: 13.02.2006
(51) Int. Cl.: A61K 31/519, A61K 31/616, A61K 9/16, A61K 9/24, A61K 9/32, A61K 9/36, A61K 9/48, A61P 7/02

(54) **Dipyridamole extended-release formulations and process for preparing same**
Dipyridamol enthaltende Zusammensetzungen mit verlängerter Freisetzung und Verfahren zu deren Herstellung
Compositions de dipyridamole à libération prolongée et leur procédé de préparation

(30) Priority: 09.02.2006 US 772257 P
(43) Date of publication of application: 22.08.2007
(73) Proprietor: TEVA PHARMACEUTICAL INDUSTRIES LTD., 49131 Petah Tiqva (IL)
(72) Inventor: Leibovici, Minutza, Natanya 42312 (IL); Kanari, Itamar, Kefar Sava 44235 (IL); Fox, Michael, Tel Aviv 67291 (IL)
(74) Representative: Nachshen, Neil Jacob

(56) References cited:
- US-A- 4 427 648
- US-A- 4 578 264
- US-A- 4 609 542
- US-A- 6 015 577

## Description

### Field of the Invention

The invention encompasses pharmaceutical formulations of extended-release formulations of dipyridamole and immediate release formulations of aspirin, and the methods of making the same.

### Background of the Invention

Dipyridamole (2,6-bis-(diethanolamino)-4,8-dipiperidino-(5,4-d)-pyrimidine) is clinically used as an active substance with antithrombotic and antiaggregatory activity.

An extended release form of administration of dipyridamole would apparently have the advantage of facilitating a reduction in the number of administrations per day, which would supposedly lead to better patient compliance. This is of significant importance with regard to long-term medication. Another advantage is that delayed resorption would reportedly lead to more uniform levels in the blood, thus avoiding or minimizing blood level peaks, which can apparently cause deleterious side effects, and avoiding sub-therapeutic levels of active substance, that can occur with instantaneous forms during longer dosage intervals. In other words, safety, compatibility, and effectiveness of the preparation can be increased. Due to the obvious advantages of an extended release form of dipyridamole, there have been previous efforts to develop such a form.

However, the physical and biochemical properties of dipyridamole would seem to be completely unsuitable for the typical development of an extended release form. The biological half-life of dipyridamole is relatively short, existing levels in the blood reportedly drop quickly, and an uniform dipyridamole level in the blood can apparently be obtained only if the active substance is constantly resorbed. Dipyridamole is reportedly only soluble in aqueous medium in the acid range; more specifically, above a pH of 4 the substance is practically insoluble in water. This seems to mean that dipyridamole can be dissolved only in the upper gastro-intestinal tract and consequently resorbed, whereas at the higher pH values occurring in the intestinal region it remains insoluble and is not resorbed.

U.S. patent No. 4,367,217 ("the '217 patent") discloses composition where dipyridamole and carboxylic acid are combined together into spheroid particles which are surrounded with a dialysis membrane consisting essentially of acid-insoluble lacquers soluble in intestinal juices. The '217 patent teaches that "if the total dose is divided into hundreds of independent, small, sustained release forms, then a statistically uniform, largely consistent passage of this sustained release form through the gastro-intestinal tract is provided." The '217 patent goes on to explain that "the effects of the differences in the pH gradient and the gastro-intestinal motility of individual patients on the dipyridamole blood level behavior are thereby largely compensated for" and that "realization of the principle of a certain pH-dependent control of release therefore necessitates in the case of dipyridamole the use of spheroid particles such as rounded granulates or pellets."

It is known that acetylsalicylic acid, being an inhibitory substance, counteracts the aggregation of human blood platelets. It has been reported that acetylsalicylic acid inhibits the enzyme cyclooxygenase in the blood platelets and thus inhibits the biosynthesis of the aggregation-promoting thromboxane A₂. As the dosage increases the antithrombotic activity of acetylsalicylic acid increases, but also at the same time its inhibitory effect on the cyclooxygenase of the blood vessel walls is increased, which indirectly has a negative influence on the synthesis of the aggregation-inhibiting substance prostacyclin. It has been suggested that the lowest possible dose of acetylsalicylic acid be used. See, Lancet, III (1979) 1213, Prostaglandins and Medicine 4 (1980) 439. On the other hand, it has been recommended that higher doses be used since, as the dosage increases, the antithrombotic activity of acetylsalicylic acid is increased even if the biosynthesis of prostacyclin and thromboxane is already inhibited. See, Prostaglandins, Leukotrienes and Medicine 12 (1983).

U.S. patent No. 4,694,024 ("the '024 patent") discloses that "surprisingly" it was found that administration, successively with a time interval, of dipyridamole and O-acetylsalicylic acid in a particular sequence with a time interval made "an extremely great improvement in the therapy of diseases which are caused or characterized by impaired blood functions or impaired constituents of blood." The '024 patent states that "[t]he combination [of] products according to the invention make it possible for the pyrimido-pyrimidine component [dipyridamole] to be released (be bioavailable) first, *i.e.* before the acetylsalicylic acid." This was based upon the observation that "the simultaneous administration of pyrimidopyrimidines, such as dipyridamole, and acetylsalicylic acid leads only to an insignificantly more potent action than is obtained on administration of acetylsalicylic acid alone." Furthermore, the '024 patent states that "the therapeutic effect cannot be increased further by increasing the relative acetylsalicylic acid weight content in the combination according to the invention to values above 2," e.g., dipyridamole to aspirin ratio of 1:2.

U.S. patent No. 6,015,577 discloses that "a combination of dipyridamole and/or mopidamol or the physiologically acceptable salts thereof and acetylsalicylic acid or the physiologically acceptable salts thereof, containing the two components in a weight ratio of more than 4.5, preferably more than 5, and which releases the two components simultaneously in the gastrointestinal tract significantly reduces or prevents clotting and at the same time will dissolve any clot already present more rapidly than would occur through natural thrombolysis." The `577 patent also states that "[i]n many cases ... it may even be advantageous if ... first the acetylsalicylic acid were released, followed by pyrimido-pyrimidine at a later stage in the gastrointestinal tract." In order to prepare a dipyridamole granulate according to the invention, dipyridamole is mixed, for example, with an organic edible acid such as fumaric, tartaric, citric, succinic or malic acid and with binders and/or adhesives such as polyvinylpyrrolidone, then a lubricant such as magnesium stearate is added, the mixture is compressed, e.g. using a roller compactor, and then broken up into granules, e.g. using a dry granulating apparatus with an adjoining screening mechanism.

U.S. patent No. 4, 578, 264 discloses modified release dipyridamole formulations in which dipyridamole and an acid, such as citric or tartaric acid, are granulated together to form a homogeneous mixture that is compressed into a tablet form.

Currently, efforts have been directed to developing delayed release dipyridamole formulations that can withstand the acidity of the stomach and release in the basic environment of the intestines. The present invention provides such a formulation either alone or in combination with an immediate release acetylsalicylic acid formulation. The dosage forms of the invention are significantly different to those described in U.S. patent Nos. 4,367,217 and 6,015,577 in at least that the dipyridamole formulations of the present invention is in the form of compressed tablets, preferably mini tablets, not granules, spherical particles or otherwise similar, and that preferably the tablets are composed of a mixture of separately processed dipyridamole and carboxylic acid,

### Summary of the Invention

One embodiment of the invention encompasses dipyridamole formulations comprising an extended release formulation of dipyridamole and a pharmaceutically acceptable carboxylic acid, wherein the extended release formulation is in a mini-tablet solid form having a diameter of about 1.5 mm to about 3 mm. The dipyridamole formulation may further comprise an immediate-release acetylsalicylic acid formulation, wherein the acetylsalicylic acid formulation is coated with an immediate release coating.

Another embodiment of the invention encompasses dipyridamole formulations comprising an extended release formulation of dipyridamole, at least one pharmaceutically acceptable excipient, and at least one carboxylic acid; and an immediate release formulation of acetylsalicylic acid and at least one pharmaceutically acceptable excipient, wherein the extended release formulation is formed in the shape of a mini-tablet and the extended release and immediate release formulations are combined in a capsule. The extended release formulation may have an extended release coating that controls the release of dipyridamole.

Preferably, in the dipyridamole formulation, the mini-tablet has a diameter of about 1.5 mm to about 3 mm. More preferably, the mini-tablet has a diameter of about 1.8 mm to about 2.2 mm.

In the dipyridamole formulation the ratio of carboxylic acid to dipyridamole may be about 1:10 to about 10:1 by weight. The extended release coating may be present in an amount of about 10% to about 20% by weight of the mini-tablet. Preferably, the extended release coating is present in an amount of about 13% to about 15% by weight of the mini-tablet.

In yet another embodiment, about 20% to about 35% by weight of the dipyridamole of the formulation dissolves after being mixed in an Apparatus USP (such as Apparatus USP I (basket)) for 1 hour in 900 ml of 0.1 N HCl. Alternatively, about 45% to about 55% by weight of the dipyridamole of the formulation dissolves after being mixed in an Apparatus USP (such as Apparatus USP I (basket)) for 1 hour in 900 ml of 0.1 N HCl and for 1 hour in 900 ml of phosphate buffer having a pH of 5.5.

Another embodiment of the invention encompasses a process for making a dipyridamole formulation comprising mixing dipyridamole and at least one carboxylic acid, preferably, the dipyridamole and the carboxylic acid are separately formulated as separate granulates before mixing; shaping the mixture into a mini-tablet; coating the mini-tablet with an extended release coating; forming an immediate release formulation of acetylsalicylic acid; and combining the coated mini-tablet and the immediate release formulation. The coated mini-tablet and the immediate release formulation may be combined into a capsule. In the process, the extended release coating is present in an amount of about 10% to about 20% by weight of the mini-tablet. Preferably, the extended release coating is present in an amount of about 13% to about 15% by weight of the mini-tablet. The mini-tablet has a diameter of about 1.5 mm to about 3 mm, and preferably, the mini-tablet has a diameter of about 1.8 mm to about 2.2 mm.

Preferably, at least one of dipyridamole or the acid is granulated, while the other could be in a powder form. More preferably, both are granulated, and most preferably, each of the dipyridamole or acid is granulated separately.

Preferably, the dipyridamole-comprising granules contain dipyridamole in an amount of at least about 70% by weight, more preferably at least about 80%, and most preferably at least about 90%. Preferably, the acid-comprising granules contain the carboxylic acid in an amount of at least about 70% by weight, more preferably at least about 80%, and most preferably at least about 90%.

According to an embodiment of the invention, dipyridamole and/or acid granules also contain at least one filler and at least one binder. Other excipients could also be used in the formulation, e.g. plasticizers and lubricants. The additional excipients could be present in the granules or extra-granularly.

According to a preferred embodiment, acid-containing granules are coated before mixing with the dipyridamole-containing granules. According to another preferred embodiment, dipyridamole-containing granules are coated before mixing with the acid-containing granules. Preferably granules of both components are coated before they are mixed prior to their being formed into tablets. Suitable coatings include water-soluble material, such as PVP, HPMC, or Opadry^{®}. Particularly preferred coatings are Opadry^{®} or Sepifilm®. Opadry® are commercially available powder mixes available from Colorcon for preparing coating dispersions comprised of hypromellose, polyethylene glycol, talc and titanium dioxide. Sepifilm® LP grade is comprised of hydroxypropylmethyl cellulose, microcrystalline cellulose, and stearic acid and is manufactured by SEPPIC Inc. (Fairfield, New Jersey 07004). The dispersions used for coating the acid-containing granules are preferably organic solutions or dispersions of the coating materials. Preferably, the solutions are ethanol or isopropyl alcohol.

Yet another embodiment encompasses a process for making a dipyridamole formulation, wherein about 20% to about 35% of the dipyridamole of the capsule dissolves after being mixed in an Apparatus USP (such as Apparatus USP I (basket)) for 1 hour in 900 ml of 0.1 N HCl. Alternatively, about 45% to about 55% by weight of the dipyridamole of the capsule dissolves after being mixed in an Apparatus USP (such as Apparatus USP I (basket)) for 1 hour in 900 ml of 0.1 N HCl and for 1 hour in 900 ml of phosphate buffer having a pH of 5.5.

### Detailed Description of the Invention

The invention encompasses a formulation comprising extended release formulation of dipyridamole and a pharmaceutically acceptable carboxylic acid, wherein the extended release formulation is in a tablet solid form having a diameter of about 1.5 mm and about 3 mm. The formulation may further comprise an immediate-release acetylsalicylic acid formulation wherein the acetylsalicylic acid formulation is coated with an immediate release coating.

Not to be limited by theory, but it is believed that the formulations of the invention maintain the dipyridamole and acetylsalicylic acid formulations separate to promote dipyridamole stability, which is known to degrade over time upon storage. In particular, the extended release formulation combines dipyridamole and at least one organic acid, such that the organic acid promotes the dissolution of the dipyridamole in the gastrointestinal environment.

In another embodiment, the invention encompasses a formulation comprising an extended release formulation of dipyridamole, at least one pharmaceutically acceptable excipient, and at least one carboxylic acid wherein the extended release formulation is formed in the shape of a mini-tablet; and an immediate release formulation of acetylsalicylic acid and at least one pharmaceutically acceptable excipient, wherein the extended release and immediate release formulations are combined in a capsule. Optionally, the dipyridamole and the organic acid of the extended release formulation may be granulated jointly or separately. The formulation may further comprise coating the extended release formulation having a coating that delays the release of dipyridamole.

As used herein unless otherwise stated, the term "mini-tablet" refers to a formulation in the shape of a tablet having a diameter of about 1.5 mm to about 3 mm. Preferably, the term "mini-tablet" refers to a tablet having a diameter of about 1.8 mm to about 2.2 mm.

In yet another embodiment, the invention encompasses a process for making a formulation comprising forming an extended release formulation of dipyridamole and at least one carboxylic acid; shaping the extended release formulation into a mini-tablet; forming an immediate release formulation of acetylsalicylic acid; and combining the extended release formulation and the immediate release formulation. Optionally, the extended release formulation and the immediate release formulation are combined into a capsule.

The dipyridamole of the extended release formulation may be dipyridamole or any pharmaceutically acceptable salt thereof. Suitable dipyridamole includes, but is not limited to, salts, solvates, anhydrates, hydrates, polymorphs, or amorphous forms. The dipyridamole may be in granulated or powdered form. If granulated, then the dipyridamole is present in an amount of about at least about 70% by weight of the granule. Preferably, the dipyridamole is present in an amount of about at least 80% by weight of the granule and more preferably, in an amount of at least 90% by weight.

Although dipyridamole is practically insoluble at a pH above 4, in vitro release tests with artificial intestinal juices having a pH of 6 to 7 have shown that dipyridamole diffuses out of the coated extended release forms. The organic acid buffers intestinal juices after penetration into the extended release form. Despite the intestinal juices surrounding the extended release form, an acidic medium prevails within the extended release form. Thus, the dipyridamole can be dissolved and diffused outwards and as the dipyridamole is resorbed, more is released continuously into the intestinal tract.

Typically, the organic acids used in the formulations of the invention are hydrophilic and capable of maintaining a pH where the dipyridamole is soluble. Preferably, the pH is about 4 or less. For example, hydrophilic acids include those having a at least one hydroxyl group and/or carbonyl group. Typically, the organic acid has a pKa of about 4.2 or less. Preferred organic acids include carboxylic acids including, but not limited to, fumaric, tartaric, citric, succinic, adipic, or malic acid. The organic acid is present in an amount sufficient to maintain a pH capable of dissolving the dipyridamole. Typically, in the extended release formulation the ratio of organic acid to dipyridamole is about 10:1 to about 1:10 by weight. Alternatively, in the extended release formulation the molar ratio of the acid to dipyridamole is about 1:2 to about 2:1. Preferably, the ratio of amount of acid is about 30% to about 60% by weight of the extended release formulation.

The organic acid may be in the form of a granule. When present as a granule, the organic acid is present in an amount of at least about 70% by weight of the granule. Preferably, the organic acid is present in an amount of at least about 80% and more preferably, in an amount of at least about 90% by weight of the granule. The organic acid may be combined with at least one excipient, such as those described below.

The tablets of the invention comprised of granules of dipyridamole and/or organic acid may have an extended release coating. Extended release coatings are coatings that extend or "slow down" the release of dipyridamole and/or organic acid. Preferably, the extended release coating are based on polymers that resist dissolving in the gastric juices of the stomach and are more soluble at the pH normally found in the intestine. These polymers are often referred to as enteric (coating) polymers, as they are usually used to prevent dissolution of the active material before reaching the intestine. In the compositions of the invention, enteric polymers are used to form an extended release coating wherein a certain amount of the active material is dissolved in the stomach and continues to be released at a defined rate on reaching the intestine. This is preferably achieved by controlling the amount of coating deposited on the tablet so that even in acid conditions the coating is, for example, "thin" enough to allow a defined amount of drug to be released from the tablet even at the acid conditions of the stomach. Most preferably the extended release coating will be one that combines two or more enteric polymers each having a characteristic pH at which they begin to dissolve. Typically, the extended release coating comprises methacrylic polymers, plasticizers, water, and a solution of ammonia, preferably a strong ammonia solution. In one embodiment, the strong ammonia solution is a solution of NH₃ containing not less than 27.0% and not more then 31.0% (w/w) of NH₃ (see USP 23). Extended release coatings include, but are not limited to, known extended release polymers and hydrophobic agents used in pharmaceutical formulations. More preferred extended release coatings, however, are enteric coating polymers including, but not limited to, at least one of methacrylic acid - ethyl acrylate copolymer, methacrylic acid - methyl methacrylate copolymer (Eudragit^{®} S), hydroxypropyl methylcellulose phthalate, cellulose acetate phthalate, ethylcellulose phthalate, hydroxypropylmethylcellulose succinate, or cellulose acetate succinate. A preferred extended release coating comprises a mixture of Eudragit^{®} S-100 (Methacrylic Acid Copolymer, Type B NF) and Eudragit^{®} L-100 (methacrylic acid copolymer, Type A NF), plasticized with triethylcitrate.

The amount of extended release coating applied to the dipyridamole formulation will depend upon the time required for dissolution and release of the dipyridamole. When using a mini-tablet and a coating of a 1:1:1 ratio by weight of Eudragit^{®} S-100, triethylcitrate, and Eudragit^{®} L-100, typical amounts of coating are about 10% to about 20% by weight of the mini-tablet. Preferably, the amount of extended release coating is about 13% to about 17% by weight of the mini-tablet.

Selection of excipients and the amounts to use can be readily determined by an experienced formulation scientist in view of standard procedures and reference works known in the art.

Excipients used in the formulations of the invention include, but are not limited to, binders, diluent, disintegrants, lubricants, sweetening agent, coloring agent, flavoring agent, or plasticizer.

Suitable binders for the formulation include, but are not limited to, acacia, alginic acid, carbomer copolymer, carbomer interpolymer, copovidone, microcrystalline cellulose, dextrin, ethylcellulose, gelatin, glucose (liquid), guar gum, hydroxypropyl cellulose, maltose, methylcellulose, polyethylene oxide, polyvinylpyrrolidone, povidone, starch, or sodium carboxymethylcellulose. Preferred binders include polyvinylpyrrolidone or copovidone. Typically, the binder is present in an amount of about 4% by weight of the mini-tablet.

Suitable excipients used in the formulation include, but are not limited to, microcrystalline cellulose (avicel), lactose, calcium phosphate, etc.

Suitable lubricants used in the formulation include, but are not limited to, calcium stearate, glyceryl behenate, magnesium stearate, polyethylene glycol, sodium stearyl fumarate, stearic acid, talc, or zinc stearate. Preferred lubricants include magnesium stearate or stearic acid. Typically, the lubricant is present in an amount of about 1.2% by weight of the mini-tablet. In the acetylsalicylic acid granulate, the lubricant is present in an amount of about 2% by weight of the acetylsalicylic acid formulation.

Suitable disintegrants used in the formulation include, but are not limited to, alginic acid, microcrystalline cellulose, croscarmellose sodium, crospovidone, maltose, polacrilin potassium, sodium starch glycolate, or starch. Preferably, the disintegrant is crospovidone. Typically, the disintegrant is present in an amount of about 2% by weight of the acetylsalicylate acid formulation.

Suitable diluent used in the formulation include, but are not limited to, calcium carbonate, calcium phosphate (dibasic and/or tribasic), calcium sulfate, microcrystalline cellulose, dextrates, dextrin, dextrose excipient, fructose, kaolin, lactitol, lactose (anhydrous and/or monohydrate), maltose, mannitol, microcrystalline cellulose, sorbitol, starch, or sucrose. Preferably, the diluent is lactose or microcrystalline cellulose. The diluent is present in an amount of about 12% by weight of the mini-tablet. Alternatively, the diluent is present in an amount of about 36% by weight of the acetylsalicylic acid formulation.

Suitable plasticizers used in the formulation include, but are not limited to, acetyltributyl citrate, acetyltriethyl citrate, castor oil, diacetylated monoglycerides, dibutyl sebacate, diethyl phthalate, glycerin, polyethylene glycol, triacetin, tributyl citrate, or triethyl citrate. Preferably, the plasticizer is triethyl citrate. Typically, the plasticizer is present in an amount of about 27% by weight of the extended release coating.

Suitable glidants used in the formulation include, but are not limited to, talc, kaolin, glycerol monostearate, silicic acid, magnesium stearate, or titanium dioxide.

The immediate release formulation includes acetylsalicylic acid which may be in the form of a pharmacologically acceptable salt. Pharmacologically acceptable salts of acetylsalicylic acid are those with pharmacologically acceptable cations such as metal cations, ammonium, amine cations, or quaternary ammonium cations. Metal cations include, but are not limited to, alkali metals, such as lithium, sodium and potassium, and of the alkaline earth metals, such as magnesium and calcium. It is also possible to use cationic forms of other metals, such as aluminum, zinc, or iron. Pharmacologically acceptable amine cations include, but are not limited to, those of primary, secondary, or tertiary amines. Amine cations include alkylamines, such as methyl-, dimethyl-, trimethyl-, ethyl-, dibutyl-, triisopropyl-, N-methylhexyl-, benzyl-, β.-phenylethylamine, ethylenediamine, diethylenetriamine, piperidine, morpholine, piperazine, mono-, di- and triethanolamine, ethyldiethanolamine, or N-butylethanolamine. Other suitable amine salts include basic amine salts of lysine and arginine. Examples of suitable pharmacologically acceptable quaternary ammonium cations include, but are not limited to, tetramethylammonium, tetraethylammonium, or benzyltrimethylammonium.

Immediate release coatings include coatings that dissolve quickly to release the acetylsalicylic acid include, but are not limited to, gastrosoluble compositions for film-coating of moisture sensitive solid particles such as the commercially available Sepifilm^{®} LP grade, by SEPPIC Inc. (Fairfield, NJ 07004) which is comprised of HPMC, MCC and stearic acid. Typically, the immediate release coating is present in an amount of about 2% to about 5% by weight of the acetylsalicylic acid formulation. Preferably, the immediate release coating is present in an amount of about 3% to about 4% by weight of the acetylsalicylic acid formulation.

In the final formulation, the weight ratio of dipyridamole to acetylsalicylic acid is typically about 8 to about 1. For example, in one embodiment, the dipyridamole is present in 200 mg and the acetyl salicylic acid is present in 25 mg. Although it is not a departure from the scope of the invention if the ratio is different as it is the novel dipyridamole formulation in combination with a dose of acetylsalicylic acid that is focused upon.

The invention also encompasses processes for making an extended release formulation of dipyridamole comprising mixing dipyridamole and at least one binder and/or other excipient into a mixture; wet granulating the mixture with a first granulating solution; drying and milling the dipyridamole granulate; mixing at least one organic acid with at least one excipient (*e.g*., binder); wet granulating the mixture with a second granulating solution; drying and milling the organic acid granulate; and mixing the dipyridamole granulate and the organic acid granulate and compressing the mixture into mini-tablets. Optionally, the process further comprises coating the dipyridamole granulate with an extended release coating before mixing the granulates. Optionally, the process further comprises coating the organic acid granulate with an extended release coating before mixing the granulates. Alternatively, the dipyridamole granulate and/or the organic acid granulate may have an immediate release coating. The process may further comprise coating the mini-tablets with an extended release coating preferably based on enteric polymers.

The process may further comprise mixing acetylsalicylic acid and at least one diluent, disintegrant, or lubricant; compressing the mixture into tablets; coating the tablets with an immediate release coating; and filling a capsule with a mixture of the mini-tablets and the coated acetylsalicylic acid formulation.

The skilled artisan with no or little experimentation can easily determine the methods, parameters, and equipment for mixing the ingredients or wet granulating the ingredients. The skilled artisan will also be able to determine the conditions for performing the drying and milling steps using equipment commonly available in the art. As the skilled artisan knows, conditions such as amount of ingredients, temperature, and/or amount of wetness, among others, will affect the conditions necessary to carry out the process. For example, mixing of the organic acid and a pharmaceutically acceptable excipient can be performed using a high sheer mixer.

The coating process can be carried out using equipment commonly used for coating granulates or tablets. For example, the coating may be performed using a Glatt fluidized bed coating machine using a bottom spray Wurster system.

The first and second granulating solvent preferably include at least one of water, ethanol, or isopropanol, optionally having dissolved therein a binder. Typically, when a binder is used in the granulation solvent, the binder is dissolved in the water and/or ethanol. Suitable binders used in the granulating solvent are those described above. Preferably, the binder used in the granulating solution is at least one of polyvinylpyrrolidone or copovidone.

Dissolution studies of the capsules of the invention demonstrated that the dipyridamole in the form of mini-tablets coated with enteric coating diffusion layer was bio-equivalent to the commercially available dipyridamole pellets (Aggrenox ® by Boehringer Ingelheim). The dipyridamole in the samples of the invention was in the form of mini-tablets. In contrast, the dipyridamole of the commercially available sample was in the form of pellets.

For example, capsules filled with the mini-tablets of the invention were tested for dissolution of dipyridamole. The method used an Apparatus USP I (Basket), mixing at a speed of 100 rpm in a first medium (I) for 1 hour in 900 ml 0.1 N HCI; samples of dissolution medium were taken at 1 hour and analyzed; and a second medium (II) for 1 to 7 hours in 900 ml phosphate buffer having a pH of 5.5. Samples of dissolution medium were taken at 1, 2, 5, and 7 hours and analyzed.

The dipyridamole of the invention in the form of mini-tablets coated with an extended release coating had a similar dissolution profile to the commercially available dipyridamole pellets. The percent amount of dipyridamole dissolved in tablets having 13% by weight of extended release coating was 28% in medium (I) and 49% (1+1 hour), 63% (2+1 hours), 83% (4 +1 hours), and 89 (7+1 hours) in medium (II). In contrast, the percent amount of dipyridamole dissolved from Aggrenox® was 21% in medium (I) and 48% (1+1 hour), 63% (2+1 hours), 84% (5+1 hours), and 90% (7+1 hours) in medium (II). Example 2 further summarizes the results of dissolution testing.

The hard capsules made using the compositions of the invention were found to be bioequivalent to Aggrenox®. The pharmacokinetics of hard capsules of the invention made with dipyridamole mini-tablets coated with an extended release coating in a quantities of 13% (Test A) and 17% (Test B) by weight of the mini-tablet were compared to that of Aggrenox®. Subjects were tested either in a fasting state or after being fed. The ratio of Cₘₐₓ of tablets of Test A/Aggrenox® was 90.77 (fast) and 105.74 (fed) and the ratio of the AUC results was 101.66 (fast) and 84.85 (fed). The Cₘₐₓ of tablets of Test B/Aggrenox® was 85.36 (fast) and 92.32 (fed) and the AUC was 95.96 (fast) and 88.78 (fed).

### Examples

### Example 1: Dipyridamole Mini-Tablets (200 mg Dipyridamole per capsule)

The dipyridamole granulate was prepared by mixing dipyridamole (200 mg/72 mini-tablets) and microcrystalline cellulose (Avicel® PH 101, 10 mg/72 mini-tablets) in a high sheer mixer and then preparing a wet granulate using a granulation solution of copovidone (Plasdone® S-630, 10 mg/72 mini-tablets) and alcohol. The wet dipyridamole granulate was dried in a fluid bed drier and then milled.

The tartaric acid granulate was prepared by mixing in a high sheer mixer, tartaric acid (214 mg/72 mini-tablets) and microcrystalline cellulose (Avicel^{®} PH 101, 10 mg/72 mini-tablets) with a granulation solution of copovidone (Plasdone^{®} S-630, 10 mg/72 mini-tablets) and alcohol. The wet tartaric acid granulate was dried in a fluid bed drier and then milled.

The dipyridamole granulate (220 mg/72 mini-tablets), the tartaric acid granulate (234 mg/72 mini-tablets), Avicel® PH 102 (40 mg/72 mini-tablets), and magnesium stearate (6 mg/72 mini-tablets) were mixed in a blender. The mixture was then compressed into mini-tablets having a diameter of 1.8 to 2.2 mm using a rotary tabletting press machine.

Separately, an extended release coating dispersion based on enteric polymers was made by mixing, talc (22.4 mg/72 mini-tablets), Eudragit^{®} S-100 (methacrylic acid copolymer, Type B NF, 32.5 mg/72 mini-tablets), triethylcitrate (32.6 mg/72 mini-tablets), Eudragit^{®}L-100 (methacrylic acid copolymer, Type A NF, 32.5 mg/72 mini-tablets), purified water, and strong ammonia solution USP.

The mini-tablets were coated with the extended release coating based on enteric polymers. Samples were taken having differing percentages of coating w/w as compared with uncoated cores, see the results in Table 1.

Acetylsalicylic acid tablets were prepared by mixing acetylsalicylic acid (25 mg/tablet), lactose (17.5 mg/tablet), microcrystalline cellulose (Avicel PH 102, 5.5 mg/tablet), stearic acid (1 mg/tablet), and crospovidone (1 mg/tablet). The mixture was compressed into tablets having a diameter of 4 to 8 mm. Separately, an immediate release coating was made by mixing Sepifilm LP 761 (2 mg/tablet) and purified water, which was used to coat the acetylsalicylic acid tablets.

Capsules No. 0 or 00 were filled with a tablet having 25 mg of acetylsalicylic acid and 70 to 74 mini-tablets of dipyridamole. The capsules may be filled with multiple tablets to obtain quantitative amounts of 25 mg of acetylsalicylic acid and 200 mg of dipyridamole per capsule.

### Example 2: Capsule Dissolution Testing

Filled capsules of Example 1 were tested for dissolution of dipyridamole extended-release mini-tablets. The method used an Apparatus USP I (Basket), mixing at a speed of 100 rpm in 900 ml 0.1 N HCI - medium (I) for 1 hour, samples of dissolution medium were taken at 1 hour and analyzed and then in 900 ml phosphate buffer having a pH of 5.5 - medium (II) for 1 to 7 hours. Samples of the dissolution medium were taken at 1, 2, 5, and 7 hours and analyzed.

Table 1 summarizes the dissolution profile for the hard gelatin capsules containing 200 mg dipyridamole versus Aggrenox® capsules manufactured by Boehringer Ingelheim. The dipyridamole in the samples of the invention is in the form of mini-tablets. In contrast, the dipyridamole of the commercially available sample is in the form of pellets.

| Table 1. Dissolution Results | | | | | | |
|---|---|---|---|---|---|---|
| Sample No. | Coating (% w/w) | % Dipyridamole dissolved Medium I (1 h) | % of Total Dipyridamole dissolved in both media (time in Medium II) | | | |
| | | | 1(+1) Hour | 2 (+1) Hours | 5 (+1) Hours | 7 (+1) Hours |
| 1 | 20 | 6 | 25 | 45 | 72 | 80 |
| 2 | 18 | 9 | 24 | 40 | 66 | 74 |
| 3 | 17 | 11 | 30 | 47 | 73 | 80 |
| 4 | 15 | 23 | 43 | 56 | 75 | 80 |
| 5 | 13 | 28 | 49 | 63 | 83 | 89 |
| Aggrenox | NA | 21 | 48 | 63 | 84 | 90 |

The dipyridamole in the form of mini-tablets coated with an enteric-coating diffusion layer has a similar dissolution profile to the dipyridamole pellets. The results of a bio-equivalence study of the hard gelatin capsules and Aggrenox is summarized in Table 2. The hard capsules of the test had dipyridamole mini-tablets coated with an enteric coating in a quantities of 13% (Test A) and 17% (Test B) by weight of the mini-tablet.

| Table 2. Bio-equivalency Testing | | | | |
|---|---|---|---|---|
| Test | Test A/ Aggrenox | | Test B/ Aggrenox | |
| | Fast | Fed | Fast | Fed |
| Cmax | 90.77 | 105.74 | 85.36 | 92.32 |
| AUC | 101.66 | 84.85 | 95.96 | 88.78 |

Dipyridamole in the form of mini-tablets coated with enteric coating diffusion layer was bio-equivalent to the commercially available dipyridamole pellets (Aggrenox by Boehringer Ingelheim). Thus, going back to the dissolution profiles it can be seen that it is preferred that after 1 hour in 900 ml 0.1 N HCl from about 10% to about 32% by weight of the dipyridamole is dissolved. And that after a subsequent hour in 900 ml phosphate buffer having a pH of 5.5, about 28% to about 55% of the dipyridamole is dissolved. One can see that although there is a correlation between dissolution rate and the results of the biostudy there is not an exact congruency and thus a margin must be taken into account.

### Example 3: Based on Dipyridamole Tablets comprised of Coated Granulates

Dipyridamole extended release min-tablets are made by making a granulate. The granulate is made by mixing dipyridamole (200 mg/72 mini-tablets) and Avicel® PH 101 (10 mg/72 mini-tablets) in a high sheer mixer. A granulate is prepared by mixing the mixture with an alcoholic solution of Plasdone® S-630 (10 mg/72 mini-tablets) and water. The wet granulate is dried in a fluid bed drier and milled. Then the granules are coated with a coating suspension of Sepifilm LP 761 (22 mg/72 mini-tablets) and water, ethanol, or isopropanol.

Separately, a coated tartaric acid granulate is made by mixing in a high sheer mixer tartaric acid (214 mg/72 mini-tablets) and Avicel® PH 101 (10 mg/72 mini-tablets). A granulate is prepared by mixing the mixture with an alcoholic solution of Plasdone® S-630 (10 mg/72 mini-tablets) and alcohol. The wet granulate is dried in a fluid bed drier and milled. Then the granules are coated with a coating suspension of Sepifilm LP 761 (24 mg/72 mini-tablets) and water, ethanol, or isopropanol.

The mini-tablets are made by mixing in a blender the coated dipyridamole granulate (242 mg), coated tartaric acid granulate (258 mg), Avicel® PH 102, and magnesium stearate (6 mg). The mixture is compressed into mini-tablets with a diameter of about 1.8 mm to about 2.2 mm using a rotatory tabletting press machine. The mix can be made of coated dipyridamole granulate and non-coated tartaric acid granulate; non-coated dipyridamole granulate and coated tartaric acid granulate; or coated dipyridamole granulate and coated tartaric acid granulate.

The mini-tablets are coated using an extended release coating. The extended release coating is made of talc (22.4 mg/72 mini-tablets), Eudragit® S-100 (32.5 mg/72 mini-tablets), triethyl citrate (32.6 mg/72 mini-tablets), Eudragit® L-100 (32.5 mg/72 mini-tablets), purified water, and strong ammonia solution.

Tablets of acetylsalicylic acid can be made as follows. Acetylsalicylic acid (Aspirin) (25 mg), lactose (17.5 mg), Avicel® PH 102 (5.5 mg), stearic acid (1 mg), and crospovidone (1 mg) are mixed and compressed into tablets having a diameter of about 4 mm to about 8 mm. Then the tablets are coated with a mixture made of Sepifilm LP 761 (2 mg) and purified water.

Capsules No. 0 or 00 can be filled with 1 acetylsalicylic acid tablet and 70 to 74 mini-tablets of dipyridamole. Each capsule will have 200 mg of dipyridamole.

## Claims

1. An extended release dipyridamole formulation comprising dipyridamole in granulate form, a pharmaceutically acceptable carboxylic acid in granulate form, and optionally one or more extra-granular excipients, wherein the formulation is in a mini-tablet solid form having a diameter of 1.5 mm to 3 mm, the ratio of carboxylic acid to dipyridamole is 1:10 to 10:1 by weight, and wherein the carboxylic acid and dipyridamole are granulated separately before being formed into the mini-tablet.

2. The dipyridamole formulation according to claim 1, comprising one or more extra-granular excipients.

3. An extended release dipyridamole formulation comprising dipyridamole, a pharmaceutically acceptable carboxylic acid in granulate form, and optionally one or morse extra-granular excipients, wherein the formulation is in a mini-tablet solid form having a diameter of 1.5 mm to 3 mm, the ratio of carboxylic acid to dipyridamole is 1:10 to 10:1 by weight, and wherein the carboxylic acid is granulated before being formed into the mini-tablet.

4. The dipyridamole formulation according to claim 3, comprising one or more extra-granular excipients.

5. An extended release dipyridamole formulation comprising dipyridamole in granulate form, a pharmaceutically acceptable carboxylic acid, and optionally one or more extra-granular excipients, wherein the formulation is in a mini-tablet solid form having a diameter of 1.5 mm to 3 mm, the ratio of carboxylic acid to dipyridamole is 1: 10 to 10:1 by weight, and wherein the dipyridamole is granulated before being formed into the mini-tablet.

6. The dipyridamole formulation according to claim 5, comprising one or more extra-granular excipients.

7. The dipyridamole formulation according to any preceding claim, wherein the extra-granular excipient is selected from binders, diluents, disintegrant, lubricants, sweetening agent, coloring agent, flavoring agent or plasticizer.

8. The dipyridamole formulation according to claim 7, wherein the extra-granular excipient is a diluent.

9. The dipyridamole formulation according to claim 8, wherein the diluent is selected from calcium carbonate, calcium phosphate (dibasic and/or tribasic), calcium sulfate, microcrystalline cellulose, dextrates, dextrin, dextrose excipient, fructose, kaolin, lactitol, lactose (anhydrous and/or monohydrate), maltose, mannitol, microcrystalline cellulose, sorbitol, starch, or sucrose.

10. The dipyridamole formulation according to claim 9, wherein the diluent is lactose or microcrystalline cellulose.

11. The dipyridamole formulation according to any preceding claim, further comprising an extended-release coating.

12. The dipyridamole formulation according to any preceding claim, further comprising an immediate-release acetylsalicylic acid formulation, wherein the acetylsalicylic acid formulation is coated with an immediate release coating.

13. The dipyridamole formulation according to claim 12, comprising:
an extended release formulation having dipyridamole, at least one pharmaceutically acceptable excipient, and at least one carboxylic acid; and
an immediate release formulation having acetylsalicylic acid and at least one pharmaceutically acceptable excipient,
wherein the extended release formulation is formed in the shape of a mini-tablet and the extended release and immediate release formulations are combined in a capsule.

14. The dipyridamole formulation according to claim 13, wherein the extended release formulation has an extended release coating that controls the release of dipyridamole.

15. The dipyridamole formulation according to any preceding claim, wherein the mini-tablet has a diameter of 1.8 mm to 2.2 mm.

16. The dipyridamole formulation according to claim 1, 3 or 5 wherein either or both of the carboxylic acid-containing granulate and dipyridamole-containing granulate are coated before being formed into the mini-tablet.

17. The dipyridamole formulation according to claim 16, wherein the coating is a water-soluble material.

18. The dipyridamole formulation according to claim 17, wherein the coating has hydroxypropyl methylcellulose, microcrystalline cellulose, and stearic acid.

19. The dipyridamole formulation according to claim 18, wherein the coating is an organic solution or a dispersion.

20. The dipyridamole formulation according to claim 11, wherein the extended release coating is present in an amount of 10% to 20% by weight of the mini-tablet.

21. The dipyridamole formulation according to claim 20, wherein the extended release coating has a 1:1:1 ratio by weight of methacrylic acid copolymer, type B NF; triethylcitrate; and methacrylic acid copolymer, type A NF and the coating is present in an amount of 13% to 17% by weight of the mini-tablet.

22. The dipyridamole formulation according to claim 20, wherein 10% to 32% by weight of the dipyridamole of the formulation dissolves after being mixed in an Apparatus USP I for 1 hour in 900 ml of 0.1 N HCI.

23. The dipyridamole formulation according to claim 20, wherein 28% to 55 % by weight of the dipyridamole of the formulation dissolves after being mixed in an Apparatus USP I for 1 hour in 900 ml of 0.1 N HCl and for 1 hour in 900 ml of phosphate buffer having a pH of 5.5.

24. A process for making a dipyridamole formulation comprising:
mixing dipyridamole optionally in granulate form, at least one carboxylic acid optionally in granulate form and optionally one or more extra-granular excipients;
shaping the mixture into a mini-tablet solid form having a diameter of 1.5 mm to 3 mm;
coating the mini-tablet with an extended release coating;
forming an immediate release formulation of acetylsalicylic acid; and
combining the coated mini-tablet and the immediate release formulation;
wherein at least one of the dipyridamole or carboxylic acid is present in granulate form, and wherein where both the dipyridamole and carboxylic acid are present in granulate form, the dipyridamole and the at least one carboxylic acid are granulated separately before mixing.

25. The process of claim 24, wherein the either or both of the dipyridamole granulate and the carboxylic acid granulate are coated before mixing.

26. The process of claim 25, wherein the coating has at least one water soluble substance selected from the group consisting of PVP, HPMC, and polyethylene glycol, and further optionally containing talc and/or titanium dioxide.

27. The process for making a dipyridamole formulation according to claim 25, wherein the coating is a combination of hydroxypropyl methylcellulose, microcrystalline cellulose, and stearic acid.

28. The process for making a dipyridamole formulation according to claim 24, wherein the coated mini-tablet and the immediate release formulation are combined into a capsule.

29. The process for making a dipyridamole formulation according to claim 24, wherein the extended release coating is present in an amount of 10% to 20% by weight of the mini-tablet.

30. The process for making a dipyridamole formulation according to claim 24, wherein the extended release coating has a 1:1:1 ratio by weight of methacrylic acid copolymer, type B NF; triethylcitrate; and methacrylic acid copolymer, type A NF.

31. The process for making a dipyridamole formulation according to claim 30, wherein the extended release coating is present in an amount of 13% to 17% by weight.

32. The process for making a dipyridamole formulation according to claim 24, wherein the mini-tablet has a diameter of 1.5 mm to 3 mm.

33. The process for making a dipyridamole formulation according to claim 24, wherein the mini-tablet has a diameter of 1.8 mm to 2.2 mm.

34. The process for making a dipyridamole formulation according to claim 28, wherein 10% to 32% by weight of the dipyridamole of the capsule dissolves after being mixed in an Apparatus USP I for 1 hour in 900 ml of 0.1 N HCl.

35. The process for making a dipyridamole formulation according to claim 28, wherein 28% to 55% by weight of the dipyridamole of the capsule dissolves after being mixed in an Apparatus USP I for 1 hour in 900 ml of 0.1 N HCl and for 1 hour in 900 ml of phosphate buffer having a pH of 5.5.

## Patentansprüche

1. Dipyridamolformulierung mit verlängerter Freisetzung, welche Dipyridamol in Granulatform, eine pharmazeutisch verträgliche Carbonsäure in Granulatform und optional einen oder mehrere extragranuläre Hilfsstoffe umfaßt, wobei die Formulierung in fester Form als Minitablette mit einem Durchmesser von 1,5 mm bis 3 mm vorliegt, wobei das Verhältnis von Carbonsäure zu Dipyridamol 1:10 bis 10:1, bezogen auf das Gewicht, beträgt, und wobei die Carbonsäure und das Dipyridamol getrennt voneinander granuliert werden, ehe sie zu der Minitablette geformt werden.

2. Dipyridamolformulierung nach Anspruch 1, welche einen oder mehrere extragranuläre Hilfsstoffe umfaßt.

3. Dipyridamolformulierung mit verlängerter Freisetzung, welche Dipyridamol, eine pharmazeutisch verträgliche Carbonsäure in Granulatform und optional einen oder mehrere extragranuläre Hilfsstoffe umfaßt, wobei die Formulierung in fester Form als Minitablette mit einem Durchmesser von 1,5 mm bis 3 mm vorliegt, wobei das Verhältnis von Carbonsäure zu Dipyridamol 1:10 bis 10:1, bezogen auf das Gewicht, beträgt, und wobei die Carbonsäure granuliert wird, ehe sie zu der Minitablette geformt wird.

4. Dipyridamolformulierung nach Anspruch 3, welche einen oder mehrere extragranuläre Hilfsstoffe umfaßt.

5. Dipyridamolformulierung mit verlängerter Freisetzung, welche Dipyridamol in Granulatform, eine pharmazeutisch verträgliche Carbonsäure und optional einen oder mehrere extragranuläre Hilfsstoffe umfaßt, wobei die Formulierung in fester Form als Minitablette mit einem Durchmesser von 1,5 mm bis 3 mm vorliegt, wobei das Verhältnis von Carbonsäure zu Dipyridamol 1:10 bis 10:1, bezogen auf das Gewicht, beträgt, und wobei das Dipyridamol granuliert wird, ehe es zu der Minitablette geformt wird.

6. Dipyridamolformulierung nach Anspruch 5, welche einen oder mehrere extragranuläre Hilfsstoffe umfaßt.

7. Dipyridamolformulierung nach einem der vorangegangenen Ansprüche, wobei der extragranuläre Hilfsstoff unter Bindemitteln, Verdünnungsmitteln, Sprengmitteln, Schmiermitteln, Süßungsmitteln, Färbemitteln, Geschmacksstoffen oder Weichmachern ausgewählt ist.

8. Dipyridamolformulierung nach Anspruch 7, wobei der extragranuläre Hilfsstoff ein Verdünnungsmittel ist.

9. Dipyridamolformulierung nach Anspruch 8, wobei das Verdünnungsmittel unter Calciumcarbonat, Calciumphosphat (zweibasig und/oder dreibasig), Calciumsulfat, mikrokristalliner Zellulose, Dextraten, Dextrin, Dextrosehilfsstoff, Fructose, Kaolin, Lactitol, Lactose (wasserfrei und/oder Monohydrat), Maltose, Mannitol, mikrokristalliner Zellulose, Sorbitol, Stärke oder Saccharose ausgewählt ist.

10. Dipyridamolformulierung nach Anspruch 9, wobei das Verdünnungsmittel Lactose oder mikrokristalline Zellulose ist.

11. Dipyridamolformulierung nach einem der vorangegangenen Ansprüche, welche weiterhin eine Beschichtung für verlängerte Freisetzung umfaßt.

12. Dipyridamolformulierung nach einem der vorangegangenen Ansprüche, welche weiterhin eine Acetylsalicylsäureformulierung mit sofortiger Freisetzung umfaßt, wobei die Acetylsalicylsäureformulierung mit einer Beschichtung für sofortige Freisetzung beschichtet ist.

13. Dipyridamolformulierung nach Anspruch 12, welche folgendes umfaßt:
eine Formulierung mit verlängerter Freisetzung, die Dipyridamol, wenigstens einen pharmazeutisch verträglichen Hilfsstoff und wenigstens eine Carbonsäure enthält, und eine Formulierung mit sofortiger Freisetzung, die Acetylsalicylsäure und wenigstens einen pharmazeutisch verträglichen Hilfsstoff enthält,
wobei die Formulierung mit verlängerter Freisetzung zu der Form einer Minitablette geformt ist und die Formulierungen mit verlängerter Freisetzung und sofortiger Freisetzung in einer Kapsel kombiniert sind.

14. Dipyridamolformulierung nach Anspruch 13, wobei die Formulierung mit verlängerter Freisetzung eine Beschichtung für verlängerte Freisetzung aufweist, die die Freisetzung von Dipyridamol kontrolliert.

15. Dipyridamolformulierung nach einem der vorangegangenen Ansprüche, wobei die Minitablette einen Durchmesser von 1,8 mm bis 2,2 mm hat.

16. Dipyridamolformulierung nach einem der Ansprüche 1, 3 oder 5, wobei entweder das Carbonsäure enthaltende Granulat oder das Dipyridamol enthaltende Granulat oder beide beschichtet werden, ehe sie zu der Minitablette geformt werden.

17. Dipyridamolformulierung nach Anspruch 16, wobei die Beschichtung ein wasserlösliches Material ist.

18. Dipyridamolformulierung nach Anspruch 17, wobei die Beschichtung Hydroxypropylmethylzellulose, mikrokristalline Zellulose und Stearinsäure enthält.

19. Dipyridamolformulierung nach Anspruch 18, wobei die Beschichtung eine organische Lösung oder eine Dispersion ist.

20. Dipyridamolformulierung nach Anspruch 11, wobei die Beschichtung für verlängerte Freisetzung in einer Menge von 10% bis 20%, bezogen auf das Gewicht der Minitablette, vorliegt.

21. Dipyridamolformulierung nach Anspruch 20, wobei die Beschichtung für verlängerte Freisetzung ein Gewichtsverhältnis von 1:1:1 von Methacrylsäurecopolymer, Typ B NF, Triethylcitrat und Methacrylsäurecopolymer, Typ A NF, aufweist und die Beschichtung in einer Menge von 13% bis 17%, bezogen auf das Gewicht der Minitablette, vorliegt.

22. Dipyridamolformulierung nach Anspruch 20, wobei sich 10 Gewichts-% bis 32 Gewichts-% des Dipyridamols in der Formulierung nach Mischen in einer USP 1-Vorrichtung für 1 Stunde in 900 ml 0,1 N HCl lösen.

23. Dipyridamolformulierung nach Anspruch 20, wobei sich 28 Gewichts-% bis 55 Gewichts-% des Dipyridamols in der Formulierung nach Mischen in einer USP 1-Vorrichtung für 1 Stunde in 900 ml 0,1 N HCl und für 1 Stunde in 900 ml Phosphatpuffer mit einem pH-Wert von 5,5 lösen.

24. Verfahren zum Herstellen einer Dipyridamolformulierung, welches folgendes umfaßt:
Mischen von Dipyridamol, optional in Granulatform, wenigstens einer Carbonsäure, optional in Granulatform, und optional einem oder mehreren extragranulären Hilfsstoffen,
Formen des Gemischs zu einer festen Form als Minitablette mit einem Durchmesser von 1,5 mm bis 3 mm,
Beschichten der Minitablette mit einer Beschichtung für verlängerte Freisetzung,
Bilden einer Formulierung mit sofortiger Freisetzung aus Acetylsalicylsäure und Vereinigen der beschichteten Minitablette und der Formulierung mit sofortiger Freisetzung,
wobei wenigstens entweder das Dipyridamol oder die Carbonsäure in Granulatform vorliegt und wobei, wenn sowohl das Dipyridamol als auch die Carbonsäure in Granulatform vorliegen, das Dipyridamol und die wenigstens eine Carbonsäure vor dem Mischen getrennt voneinander granuliert werden.

25. Verfahren nach Anspruch 24, wobei entweder das Dipyridamolgranulat oder das Carbonsäuregranulat oder beide vor dem Mischen beschichtet wird/werden.

26. Verfahren nach Anspruch 25, wobei die Beschichtung wenigstens eine wasserlösliche Substanz, ausgewählt aus der Gruppe, bestehend aus PVP, HPMC und Polyethylenglycol, enthält und weiterhin optional Talk und/oder Titandioxid enthält.

27. Verfahren zum Herstellen einer Dipyridamolformulierung nach Anspruch 25, wobei die Beschichtung eine Kombination aus Hydroxypropylmethylzellulose, mikrokristalliner Zellulose und Stearinsäure ist.

28. Verfahren zum Herstellen einer Dipyridamolformulierung nach Anspruch 24, wobei die beschichtete Minitablette und die Formulierung mit sofortiger Freisetzung zu einer Kapsel kombiniert werden.

29. Verfahren zum Herstellen einer Dipyridamolformulierung nach Anspruch 24, wobei die Beschichtung für verlängerte Freisetzung in einer Menge von 10% bis 20%, bezogen auf das Gewicht der Minitablette, vorliegt.

30. Verfahren zum Herstellen einer Dipyridamolformulierung nach Anspruch 24, wobei die Beschichtung für verlängerte Freisetzung ein Gewichtsverhältnis von 1:1:1 von Methacrylsäurecopolymer, Typ B NF, Triethylcitrat und Methacrylsäurecopolymer, Typ A NF, aufweist.

31. Verfahren zum Herstellen einer Dipyridamolformulierung nach Anspruch 30, wobei die Beschichtung für verlängerte Freisetzung in einer Menge von 13 Gewichts-% bis 17 Gewichts-% vorliegt.

32. Verfahren zum Herstellen einer Dipyridamolformulierung nach Anspruch 24, wobei die Minitablette einen Durchmesser von 1,5 mm bis 3 mm hat.

33. Verfahren zum Herstellen einer Dipyridamolformulierung nach Anspruch 24, wobei die Minitablette einen Durchmesser von 1,8 mm bis 2,2 mm hat.

34. Verfahren zum Herstellen einer Dipyridamolformulierung nach Anspruch 28, wobei sich 10 Gewichts-% bis 32 Gewichts-% des Dipyridamols in der Kapsel nach Mischen in einer USP I-Vorrichtung für 1 Stunde in 900 ml 0,1 N HCI lösen.

35. Verfahren zum Herstellen einer Dipyridamolformulierung nach Anspruch 28, wobei sich 28 Gewichts-% bis 55 Gewichts-% des Dipyridamols in der Kapsel nach Mischen in einer USP 1-Vorrichtung für 1 Stunde in 900 ml 0,1 N HCl und für 1 Stunde in 900 ml Phosphatpuffer mit einem pH-Wert von 5,5 lösen.

## Revendications

1. Formulation de dipyridamole à libération modifiée comprenant du dipyridamole sous forme granulée, un acide carboxylique, acceptable sur le plan pharmaceutique, sous forme granulée, et éventuellement un ou plusieurs excipients extragranulaires, dans laquelle formulation, qui est sous une forme solide de mini-comprimés ayant un diamètre compris entre 1,5 mm et 3 mm, le rapport acide carboxylique/dipyridamole est compris entre 1/10 et 10/1 en poids, et l'acide carboxylique et le dipyridamole sont granulés séparément avant d'être mis sous forme de mini-comprimés.

2. Formulation de dipyridamole selon la revendication 1, comprenant un ou plusieurs excipients extragranulaires.

3. Formulation de dipyridamole à libération modifiée comprenant du dipyridamole, un acide carboxylique, acceptable sur le plan pharmaceutique, sous forme granulée, et éventuellement un ou plusieurs excipients extragranulaires, dans laquelle formulation, qui est sous une forme solide de mini-comprimés ayant un diamètre compris entre 1,5 mm et 3 mm, le rapport acide carboxylique/dipyridamole est compris entre 1/10 et 10/1 en poids, et l'acide carboxylique est granulé avant d'être mis sous forme de mini-comprimés.

4. Formulation de dipyridamole selon la revendication 3, comprenant un ou plusieurs excipients extragranulaires.

5. Formulation de dipyridamole à libération modifiée comprenant du dipyridamole sous forme granulée, un acide carboxylique acceptable sur le plan pharmaceutique, et éventuellement un ou plusieurs excipients extragranulaires, dans laquelle formulation, qui est sous une forme solide de mini-comprimés ayant un diamètre compris entre 1,5 mm et 3 mm, le rapport acide carboxylique/dipyridamole est compris entre 1/10 et 10/1 en poids, et le dipyridamole est granulé avant d'être mis sous forme de mini-comprimés.

6. Formulation de dipyridamole selon la revendication 5, comprenant un ou plusieurs excipients extragranulaires.

7. Formulation de dipyridamole selon l'une quelconque des revendications précédentes, dans laquelle l'excipient extragranulaire est choisi dans le groupe comprenant les ligands, les diluants, les agents de délitement, les lubrifiants, les édulcorants, les agents colorants, les agents aromatisants ou les plastifiants.

8. Formulation de dipyridamole selon la revendication 7, dans laquelle l'excipient extragranulaire est un diluant.

9. Formulation de dipyridamole selon la revendication 8, dans laquelle le diluant est choisi dans le groupe comprenant le carbonate de calcium, le phosphate de calcium (dibasique et/ou tribasique), le sulfate de calcium, la cellulose microcristalline, les dextrates, la dextrine, l'excipient de dextrose, le fructose, le kaolin, le lactitol, le lactose (anhydre et/ou monohydraté), le maltose, le mannitol, la cellulose microcristalline, le sorbitol, l'amidon ou le saccharose.

10. Formulation de dipyridamole selon la revendication 9, dans laquelle le diluant est le lactose ou la cellulose microcristalline.

11. Formulation de dipyridamole selon l'une quelconque des revendications précédentes, comprenant en outre un enrobage à libération modifiée.

12. Formulation de dipyridamole selon l'une quelconque des revendications précédentes, comprenant en outre une formulation d'acide acétylsalicylique à libération immédiate, dans laquelle la formulation d'acide acétylsalicylique est entourée d'un enrobage à libération immédiate.

13. Formulation de dipyridamole selon la revendication 12, comprenant une formulation à libération modifiée contenant du dipyridamole, au moins un excipient acceptable sur le plan pharmaceutique, et au moins un acide carboxylique; et comprenant une formulation à libération immédiate contenant de l'acide acétylsalicylique et au moins un excipient acceptable sur le plan pharmaceutique, ladite formulation à libération modifiée étant mise sous forme de mini-comprimés et les formulations à libération modifiée et à libération immédiate étant combinées dans une capsule

14. Formulation de dipyridamole selon la revendication 13, dans laquelle la formulation à libération modifiée présente un enrobage à libération modifiée qui régule la libération du dipyridamole.

15. Formulation de dipyridamole selon l'une quelconque des revendications précédentes, dans laquelle les mini-comprimés présentent un diamètre compris entre 1,8 mm et 2,2 mm.

16. Formulation de dipyridamole selon la revendication 1, 3 ou 5, dans laquelle le granulat contenant de l'acide carboxylique et/ou le granulat contenant du dipyridamole sont enrobés avant d'être mis sous forme de mini-comprimés.

17. Formulation de dipyridamole selon la revendication 16, dans laquelle l'enrobage est une matière hydrosoluble.

18. Formulation de dipyridamole selon la revendication 17, dans laquelle l'enrobage contient de l'hydroxypropylméthylcellulose, de la cellulose microcristalline et de l'acide stéarique.

19. Formulation de dipyridamole selon la revendication 18, dans laquelle l'enrobage est une solution organique ou une dispersion.

20. Formulation de dipyridamole selon la revendication 11, dans laquelle l'enrobage à libération modifiée est présent à raison de 10% à 20% en poids dans les mini-comprimés.

21. Formulation de dipyridamole selon la revendication 20, dans laquelle l'enrobage à libération modifiée présente un rapport pondéral, 1/1/1, de copolymère d'acide méthacrylique, type B NF; de citrate de triéthyle; et de copolymère d'acide méthacrylique, type A NF et l'enrobage forme de 13% à 17% en poids des mini-comprimés.

22. Formulation de dipyridamole selon la revendication 20, dans laquelle 10% à 32% en poids du dipyridamole de la formulation se dissolvent après épreuve dans un appareil USP I pendant 1 heure dans 900 ml de HCI 0,1 N.

23. Formulation de dipyridamole selon la revendication 20, dans laquelle 28% à 55% en poids du dipyridamole de la formulation se dissolvent après épreuve dans un appareil USP I pendant 1 heure dans 900 ml de HCI 0,1 N et pendant 1 heure dans 900 ml de tampon phosphate ayant un pH de 5,5.

24. Procédé de fabrication d'une formulation de dipyridamole comprenant les étapes consistant à:
- mélanger le dipyridamole éventuellement sous forme granulée, au moins un acide carboxylique éventuellement sous forme granulée et éventuellement un ou plusieurs excipients extragranulaires;
- mettre le mélange sous une forme solide de mini-comprimés ayant un diamètre de 1,5 mm à 3 mm;
- entourer les mini-comprimés d'un enrobage à libération modifiée;
- former une formulation à libération immédiate d'acide acétylsalicylique; et
- combiner le mini-comprimés enrobé et la formulation à libération immédiate;
dans lequel au moins un des constituants dipyridamole ou acide carboxylique est présent sous une forme granulée, et dans lequel, lorsque le dipyridamole et l'acide carboxylique sont tous deux présents sous forme granulée, le dipyridamole et le au moins un acide carboxylique sont granulés séparément avant mélange.

25. Procédé selon la revendication 24, dans lequel le granulat de dipyridamole et le granulat d'acide carboxylique sont enrobés avant mélange.

26. Procédé selon la revendication 25, dans lequel l'enrobage a au moins une substance hydrosoluble choisie dans le groupe formé par PVP, HPMC et le polyéthylèneglycol, et contenant en outre éventuellement le talc et/ou le dioxyde de titane.

27. Procédé de fabrication d'une formulation de dipyridamole selon la revendication 25, dans lequel l'enrobage est une combinaison d'hydroxypropyl-méthylcellulose, de cellulose microcristalline et d'acide stéarique.

28. Procédé de fabrication d'une formulation de dipyridamole selon la revendication 24, dans lequel les mini-comprimés enrobés et la formulation à libération immédiate sont combinés pour former une capsule.

29. Procédé de fabrication d'une formulation de dipyridamole selon la revendication 24, dans lequel l'enrobage à libération modifiée est présent à raison de 10% à 20% en poids des mini-comprimés.

30. Procédé de fabrication d'une formulation de dipyridamole selon la revendication 24, dans lequel l'enrobage à libération modifiée possède un rapport pondéral de 1/1/1 de copolymère d'acide méthacrylique, type B NF; de citrate de triéthyle; et de copolymère d'acide méthacrylique, type A NF.

31. Procédé de fabrication d'une formulation de dipyridamole selon la revendication 30, dans lequel l'enrobage à libération modifiée est présent à raison de 13% à 17% en poids.

32. Procédé de fabrication d'une formulation de dipyridamole selon la revendication 24, dans lequel les mini-comprimés présentent un diamètre compris entre 1,5 mm à 3 mm.

33. Procédé de fabrication d'une formulation de dipyridamole selon la revendication 24, dans lequel les mini-comprimés présentent un diamètre compris entre 1,8 mm à 2,2 mm.

34. Procédé de fabrication d'une formulation de dipyridamole selon la revendication 28, dans lequel 10% à 32% en poids du dipyridamole de la capsule se dissolvent après avoir été mélangés dans un appareil USP I pendant 1 heure dans 900 ml de HCl 0,1 N.

35. Procédé de fabrication d'une formulation de dipyridamole selon la revendication 28, dans lequel 28% à 55% en poids du dipyridamole de la capsule se dissolvent après épreuve dans un appareil USP I pendant 1 heure dans 900 ml de HCl 0,1 N et pendant 1 heure dans 900 ml de tampon phosphate ayant un pH de 5,5.
